# EUROPÄISCHE PATENTANMELDUNG

(11) **EP 3 789 125 A1**
(43) Veröffentlichungstag der Anmeldung: **10.03.2021**
(21) Anmeldenummer: 19195188.8
(22) Anmeldetag: 03.09.2019
(51) Int. Cl.: B06B 1/06, G01H 11/08, A61B 8/00

(54) **IMPLANTIERBARER ULTRASCHALLWANDLER**

(71) Anmelder: BIOTRONIK SE & Co. KG, 12359 Berlin (DE)
(72) Erfinder: Bihler, Eckardt, 8406 Winterthur (CH); Johannsen, Sven, 8309 Nürensdorf (CH)
(74) Vertreter: Galander, Marcus

(57) **Zusammenfassung**

Die vorliegende Erfindung betrifft einen implantierbaren Ultraschallwandler mit einer Vorrichtung zum Erzeugen von Ultraschall, einer Vorrichtung zum Empfangen von Ultraschall, und einer Leiterplatte, wobei die Vorrichtung zum Erzeugen von Ultraschall aus einem piezoelektrischen Polymer gebildet ist, welches in die Leiterplatte integriert ist. Die vorliegende Erfindung betrifft weiterhin ein in den Körper einbringbares Medizingerät mit einem solchen Ultraschallwandler

## Beschreibung

Die vorliegende Erfindung betrifft einen implantierbaren Ultraschallwandler und ein Medizingerät mit einem solchen Ultraschallwandler.

Ultraschallwandler werden bereits in zahlreichen medizinischen Anwendungen, insbesondere bildgebenden Verfahren verwendet. Ultraschallwandler können auch für andere Aufgaben z.B. die Messungen von Schichtdicken und Abständen eingesetzt werden. Dafür bedarf es auf die jeweilige Aufgabe zugeschnittene Konzepte für den optimalen Aufbau der Wandler. Für die Anwendung innerhalb des Körpers oder auf der Haut sind besondere Anforderungen zu erfüllen.

Ultraschallwandler für Diagnostik und Messtechnik müssen einen möglichst großen Schalldruck erzeugen und gleichzeitig möglichst empfindlich und schnell detektieren können. Dieser Widerspruch macht aufwendige Signalaufarbeitung möglichst nahe am Wandler notwendig. Durch den komplexen Aufbau aus Piezokeramik, Halbleiterkomponenten und Leiterplatte sind der für Katheter und Implantate notwendigen Miniaturisierung Grenzen gesetzt.

Heute werden überwiegend Piezokeramiken aus PZT (Blei-Zirkonat-Titanat) als Ultraschallwandler eingesetzt. Diese Piezokeramiken sind kostengünstig herstellbar und weisen einen starken piezoelektrischen Effekt auf, was in großen Schallamplituden bei der Erzeugung von Schallwellen resultiert. Die Nachteile sind Limitierungen bei den Dimensionen für Strukturierung und Dicke, was die Empfindlichkeit bei der Detektion und die Signalbandbreite, sowie die Möglichkeiten zur Miniaturisierung begrenzt.

Ein weiterer Nachteil ist die schlechte akustische Anpassung an Körpergewebe, was zu hohen Energieverlusten durch Reflexion an der Grenzfläche zwischen Keramik und Körpergewebe führt. Auch angesichts des problematischen Bleigehalts sind PZT Keramiken nicht die erste Wahl für Katheter und Implantate, und nur mit zusätzlichem Aufwand in Kathetern und Implantaten verwendbar. Die resultierenden Kosten limitieren die möglichen Anwendungen.

Weitere Materialien die in Frage kommen sind Halbleiter-MEMS (CMUT - *capacitive micromachined ultrasound transducer*) und AlN. Unter den organischen Materialien mit Piezoeffekt sticht insbesondere PVDF (Polyvinylindenflourid) und sein Copolymer P(VDF-TrFE) hervor.

Der Piezoeffekt in P(VDF-TrFE) ist zwar nur 1/10 des Wertes von PZT, die akustische Impedanz von PVDF liegt jedoch mit 4.2 MPa*s/m nur wenig über der von Körpergewebe (1.6 MPa*s/m) und ist weit besser als PZT mit 30 MPa*s/m an Wasser bzw. Körpergewebe angepasst. Da PVDF in Folien mit Dicken ab 10 µm verfügbar ist, können sehr empfindliche Detektoren mit hoher Bandbreite hergestellt werden. PVDF wird daher insbesondere für Hydrophone (Ultraschalldetektoren für Unterwasseranwendungen) verwendet.

Basierend auf diesem Hintergrund ist es insbesondere eine Aufgabe der vorliegenden Erfindung, einen zuverlässigen und präzisen Ultraschallwandler zur Verfügung zu stellen, der einen vereinfachten Aufbau aufweist und im Körper eines Patienten eingesetzt werden kann, sowie entsprechende in den Körper einbringbare Medizingeräte.

Diese Aufgabe wird durch einen implantierbaren Ultraschallwandler mit den Merkmalen des Anspruchs 1 und ein Medizingerät mit den Merkmalen des Anspruchs 14 gelöst. Vorteilhafte Ausgestaltungen sind in den abhängigen Ansprüchen und der folgenden Beschreibung angegeben.

Gemäß Anspruch 1 wird ein implantierbarer Ultraschallwandler zur Verfügung gestellt. Der Ultraschallwandler weist auf:
- eine Vorrichtung zum Erzeugen von Ultraschall, hier auch als Ultraschallerzeuger bezeichnet,
- eine Vorrichtung zum Empfangen von Ultraschall, hier auch als Ultraschallempfänger bezeichnet, und
- eine Leiterplatte.

Erfindungsgemäß ist insbesondere vorgesehen, dass die Vorrichtung zum Erzeugen von Ultraschall aus einem piezoelektrischen Polymer gebildet ist, welches in die Leiterplatte integriert ist.

Der Begriff "piezoelektrisches Polymer" im Kontext der vorliegenden Erfindung bezeichnet insbesondere ein Polymer, welches durch einen piezoelektrischen Effekt charakterisiert ist, d. h. dem Auftreten einer Spannung bei elastischer Verformung.

Der Begriff "Leiterplatte" wird im Kontext der vorliegenden Erfindung in seiner dem Fachmann bekannten Bedeutung bezeichnet. Als "Leiterplatte" wird insbesondere ein Träger von elektronischen Bauteilen bezeichnet, der neben einem elektrisch isolierenden Substrat Leiterbahnen zur elektrischen Kontaktierung von elektronischen Bauteilen umfasst.

Vorteilhafterweise können durch die Trennung von Ultraschallerzeuger und Ultraschallempfänger in zwei separate Vorrichtungen der Aufbau des Ultraschallempfängers vereinfacht und unkompliziert biokompatibel ausgestaltet werden. Dies wird insbesondere durch die Integration des Ultraschallempfängers in die Leiterplatte realisiert, die selbst vorzugsweise aus einem biokompatiblen Material gefertigt ist.

Dementsprechend ist gemäß einer Ausführungsform des erfindungsgemäßen Ultraschallwandler vorgesehen, dass die Leiterplatte ein Polyimid oder ein FlüssigKristall-Polymer aufweist oder daraus besteht.

Gemäß einer Ausführungsform des erfindungsgemäßen implantierbaren Ultraschallwandlers ist vorgesehen, dass die Vorrichtung zum Empfangen von Ultraschall aus einer Vielzahl von piezoelektrischen Elementen aus dem piezoelektrischen Polymer gebildet ist, wobei die Vielzahl von piezoelektrischen Elementen in die Leiterplatte integriert sind.

Dabei können die piezoelektrischen Elemente jede beliebige Gestalt annehmen, z. B. im Wesentlichen zylinder-, würfel-, quader- oder prismaförmig.

Gemäß einer weiteren Ausführungsform des erfindungsgemäßen implantierbaren Ultraschallwandlers ist vorgesehen, dass die piezoelektrischen Elemente in einem Array in der Leiterplatte angeordnet sind, beispielsweise 3x3, 5x5, 10x10 oder 20x 50. Ein solches Array ermöglicht in vorteilhafter Weise eine ortsaufgelöste Messung des Schalldruckes, insbesondere des von Körpergeweben reflektierten Schalls.

Gemäß einer weiteren Ausführungsform des erfindungsgemäßen implantierbaren Ultraschallwandlers ist vorgesehen, dass die Leiterplatte eine Stärke oder Dicke im Bereich von 0,01 mm bis 0,1 mm aufweist.

Gemäß einer weiteren Ausführungsform des erfindungsgemäßen implantierbaren Ultraschallwandlers ist vorgesehen, dass die piezoelektrischen Elemente unabhängig voneinander eine Länge im Bereich von 0,01 mm bis 5 mm aufweisen.

Gemäß einer weiteren Ausführungsform des erfindungsgemäßen implantierbaren Ultraschallwandlers ist vorgesehen, dass die Vorrichtung zum Erzeugen von Ultraschall aus einem piezoelektrischen Material, insbesondere einer Keramik oder einem Kristall, gebildet wird, vorzugsweise eine PZT(Blei-Zirkonat-Titanat)-Keramik.

Gemäß einer weiteren Ausführungsform des erfindungsgemäßen implantierbaren Ultraschallwandlers ist vorgesehen, dass das piezoelektrische Material eine Stärke oder Dicke im Bereich von 0,1 mm bis 2 mm aufweist.

Gemäß einer weiteren Ausführungsform des erfindungsgemäßen implantierbaren Ultraschallwandlers ist vorgesehen, dass die Vorrichtung zum Erzeugen von Ultraschall an der Leiterplatte angeordnet ist.

Vorteilhafterweise ist die Vorrichtung zum Erzeugen von Ultraschall über eine Klebeschicht mit der Leiterplatte verbunden. Die Klebeschicht kann überall oder nur an bestimmten Stellen elektrisch leitfähig sein. Nicht beschränkende Beispiele für geeignete Klebstoffe umfassen Epoxid- oder Acrylharze, welche mit elektrisch leitfähigen Partikeln aus beispielsweise Metall oder Kohlenstoff, gefüllt sind. Solche Klebstoffe umfassen insbesondere bis zu 30 Vol. % an elektrisch leitfähigen Partikeln.

Gemäß einer weiteren Ausführungsform des erfindungsgemäßen implantierbaren Ultraschallwandlers ist vorgesehen, dass die Vorrichtung zum Erzeugen von Ultraschall und die Vorrichtung zum Empfangen von Ultraschall unabhängig voneinander elektrisch kontaktierbar sind. Damit kann vorteilhafter Weise der Aufbau, insbesondere die Schaltung, des erfindungsgemäßen Ultraschallwandlers vereinfacht werden, indem die Erzeugung und die Detektion in voneinander getrennten Schaltkreisen realisiert werden. Mit der Trennung von Schallerzeugung und Detektion kann ein besseres Signal-RauschVerhältnis bzw. eine höhere Empfindlichkeit bei der Detektion erreicht werden.

Gemäß einer weiteren Ausführungsform des erfindungsgemäßen implantierbaren Ultraschallwandlers ist vorgesehen, dass die Leiterplatte mindestens eine elektrische Leiterbahn umfasst, welche die Vorrichtung zum Empfangen von Ultraschall elektrisch kontaktiert.

Gemäß einer weiteren Ausführungsform des erfindungsgemäßen implantierbaren Ultraschallwandlers ist vorgesehen, dass die Vorrichtung, insbesondere das piezoelektrische Material zum Erzeugen von Ultraschall auf der Oberseite und der Unterseite eine Metallschicht aufweist. Die Metallschicht an der Oberseite kann mit Vorteil als Masse für den Ultraschallempfänger verwendet werden.

Gemäß einer weiteren Ausführungsform des erfindungsgemäßen implantierbaren Ultraschallwandlers ist vorgesehen, dass die Vorrichtung zum Erzeugen von Ultraschall mit der Oberseite an der Leiterplatte angeordnet ist, wobei die Metallschicht auf der Oberseite die Vorrichtung zum Empfangen von Ultraschall elektrisch kontaktiert, insbesondere direkt oder indirekt über eine elektrisch leitfähige Klebeschicht.

Gemäß einer alternativen Ausführungsform des erfindungsgemäßen implantierbaren Ultraschallwandlers ist vorgesehen, dass die Vorrichtung zum Erzeugen von Ultraschall an zwei einander gegenüberliegenden Seitenflächen bzw. Stirnflächen jeweils eine Metallschicht aufweist. Mit einem solcherart ausgestalteten Ultraschallwandler ist es mit Vorteil möglich, Transversallwellen in die umliegende Umgebung, z.B. Körpergewebe, einzukoppeln.

Gemäß einer weiteren Ausführungsform des erfindungsgemäßen implantierbaren Ultraschallwandlers ist vorgesehen, dass die Leiterplatte flexibel ist. Dadurch ist es mit Vorteil möglich, die Leiterplatte an die Form oder Kontur des Ultraschallerzeugers anzupassen.

Gemäß einer weiteren Ausführungsform des erfindungsgemäßen implantierbaren Ultraschallwandlers ist vorgesehen, dass das piezoelektrische Material im Wesentlichen im Form eines Hohlzylinders ausgebildet ist, wobei die Mantelseite (Oberseite) des Hohlzylinders eine Metallschicht aufweist, die den Durchgang des Hohlzylinders begrenzende Oberfläche (Unterseite) mit Metall beschichtet ist oder der Durchgang des Hohlzylinders mit Metall ausgefüllt ist, und wobei die Leiterplatte im Wesentlichen vollständig die Metallschicht auf der Mantelseite umgibt. Mit dieser Ausgestaltung des erfindungsgemäßen implantierbaren Ultraschallwandlers ist es mit Vorteil möglich, eine Zylinderwelle zu erzeugen. Damit können insbesondere Gefäß, wie Blutgefäß, per Ultraschall untersucht werden. Damit eignet sich insbesondere diese Ausgestaltung zur Verwendung in einem Katheter.

Gemäß einer weiteren Ausführungsform des erfindungsgemäßen implantierbaren Ultraschallwandlers ist vorgesehen, dass das piezoelektrische Polymer Polyvinylidenflourid oder ein Co-Polymer davon ist.

Gemäß einer weiteren Ausführungsform des erfindungsgemäßen implantierbaren Ultraschallwandlers ist vorgesehen, dass der implantierbare Ultraschallwandler zumindest teilweise mit einem biokompatiblen Lack oder Polymer, insbesondere Polydimethylsiloxan (PDMS) oder ein Polyurethan, beschichtet ist. Vorzugsweise können damit gegebenenfalls offenliegende Leiterbahnen der Leiterplatte gegen Körpergewebe oder Körperflüssigkeiten elektrisch isoliert und/oder vor Korrosion geschützt werden.

Gemäß Anspruch 14 wird ein in den Körper einbringbares, insbesondere implantierbares, Medizingerät zur Verfügung gestellt, welches den erfindungsgemäßen implantierbaren Ultraschallwandler umfasst.

In einer weiteren Ausführungsform ist das erfindungsgemäße in den Körper einbringbare, insbesondere implantierbare, Medizingerät als aktives Implantat, als Sensor, als Loop-Recorder oder als Katheter ausgebildet.

Der Begriff "Loop-Recorder" im Sinne der Erfindung bezeichnet insbesondere ein passives Implantat, welches physiologische Parameter eines Patienten misst bzw. überwacht, beispielsweise die elektrische Aktivität des Herzens.

In einer weiteren Ausführungsform ist das erfindungsgemäße in den Körper einbringbare, insbesondere implantierbare, Medizingerät als Herzschrittmacher, Kardioverter-Defibrillator, Neurostimulator oder Muskelstimulator ausgebildet.

In einer weiteren Ausführungsform ist in dem als Katheter ausgebildeten Medizingerät der erfindungsgemäße Ultraschallwandler am distalen Ende des Katheters angeordnet. Vorzugsweise ist dabei das piezoelektrische Material im Wesentlichen im Form eines Hohlzylinders ausgebildet, wobei die Mantelseite (Oberseite) des Hohlzylinders eine Metallschicht aufweist, die den Durchgang des Hohlzylinders begrenzende Oberfläche

(Unterseite) mit Metall beschichtet ist oder der Durchgang des Hohlzylinders mit Metall ausgefüllt ist, und wobei die Leiterplatte im Wesentlichen vollständig die Metallschicht auf der Mantelseite umgibt. Vorzugsweise umfasst das als Katheter ausgebildete Medizingerät weiterhin einen Katheterschlauch, einen oder mehrere Führungsdrähte sowie eine oder mehrere elektronische Komponenten, insbesondere zur Verarbeitungen von Signalen vom Ultraschallempfänger, die insbesondere auf der Leiterplatte montiert sind.

Weitere Merkmale und Vorteile der Erfindung werden nachfolgend anhand der Figurenbeschreibung von Ausführungsbeispielen erläutert. Es zeigen:

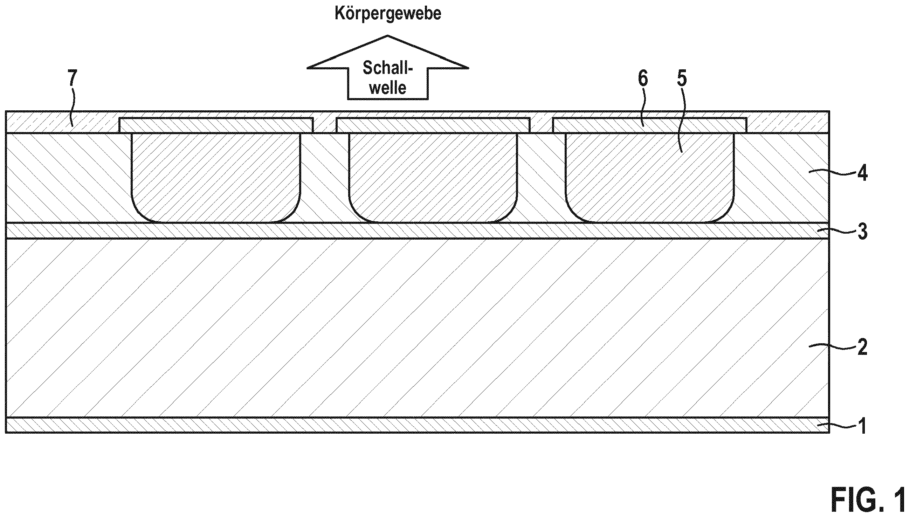

- Figuren 1 bis 6: verschiedene Ausführungsformen des erfindungsgemäßen implantierbaren Ultraschallwandlers.

Ein wesentlicher Gedanke der Erfindung ist die Abtrennung der Detektion von der Schallerzeugung in Ultraschallwandlern. Zur Detektion wird nur eine dünne Schicht verwendet, die auf einem Schallerzeuger fest verbunden ist.

Ein weiterer erfinderischer Gedanke ist die Integration der zur Detektion verwendeten piezoelektrischen Elemente in eine flexible Leiterplatte. Damit werden die Detektoren direkt elektrisch angebunden und können ohne zusätzliche Aufbau- und Verbindungstechnik direkt mit elektrischen Komponenten verbunden werden.

Die vorliegende Erfindung ermöglicht eine weitere Miniaturisierung und Vereinfachung im Aufbau mit deutlich niedrigeren Kosten, so dass weitere Anwendungen möglich werden. Ein weiterer Vorteil ist die Verwendung von bio-kompatiblen Materialien.

Insbesondere wird durch die Erfindung die Konstruktion von Ultraschallwandler für Katheter und Implantate deutlich vereinfacht und kann weiter miniaturisiert werden. Die Kosten für die Verbindungstechnik können deutlich reduziert werden.

Figur 1 zeigt eine Ausführungsform des erfindungsgemäßen implantierbaren Ultraschallwandlers. Auf einem Schallerzeuger aus einem piezoelektrischen Material 2, der auf der Oberseite 3 und der Unterseite 1 mit einer Metallschicht überzogen ist, ist eine dünne Polymerfolie 4 aufgebracht. Im einfachsten Fall ist diese Polymerfolie aus PVDF (Polyvinylidendifluorid) oder P(VDF-TrFE) (Poly(vinylidenfluoride-co-trifluoroethylen)), welche durch geeignete Polarisation piezoelektrisch gemacht wird. Vorzugsweise ist jedoch dieser Polymerfilm eine flexiblen Leiterplatte, die z.B. aus Polyimid oder LCP (Liquid Crystal Polymer) oder einem anderen Basismaterial für flexible Leiterplatten. Das hat den Vorteil, dass die flexible Leiterplatte bereits elektrische Leiterbahnen hat, die mit den Metallschichten 1, 3 und 6 auf geeignete Art verbunden sind. Durch diese Leiterbahnen werden die piezoelektrischen Materialien 2 und 5 elektrisch an eine geeignete elektrische Schaltung zur Erzeugung von Pulsen zur Schallerzeugung und zur Signalverarbeitung bei der Detektion von reflektierten Schallsignalen aus dem Körpergewebe angeschlossen. In diesem Fall wird beispielsweise das Polymer P(VDF-TrFE) 5 in geeignete Vertiefungen 5 der flexiblen Leiterplatte 4 eingebracht. Das kann entweder durch einen Wärmeprozess geschehen (P(VDF-TrFE) ist thermoplastisch und schmilzt bei ca. 150 °C) oder durch Siebdruck bzw. Rakeln einer hochviskosen Lösung von P(VDF-TrFE) in einem geeigneten Lösemittel. Vor Aufbringen der Metallisierung 6 wird mittels Koronaentladung das P(VDF-TrFE) polarisiert, um es piezoelektrisch zu machen. Danach wird die Metallstruktur zur Ableitung der Signale aufgebracht und strukturiert. Das kann mittels Sputtern und Lift-Off geschehen. Zum Schluss wird der komplette Wandler mit einem dünnen (bio-kompatiblen) Lack 7 überzogen oder mit Silikon (PDMS) 7 beschichtet, damit die Leiterbahnen 6 nicht über das Körpergewebe kurzgeschlossen werden.

Dabei haben die Komponenten des erfindungsgemäßen Ultraschallwandlers vorzugsweise folgenden Dimensionen:
- Dicke des Schallerzeugers 2: 0.1 mm bis 2 mm
- Dicke der Polymerfolie / flexible Leiterplatte 4: 0.01 mm bis 0.1 mm
- Abmessungen der piezoelektrischen Elemente 5: 0.01 mm bis 5 mm

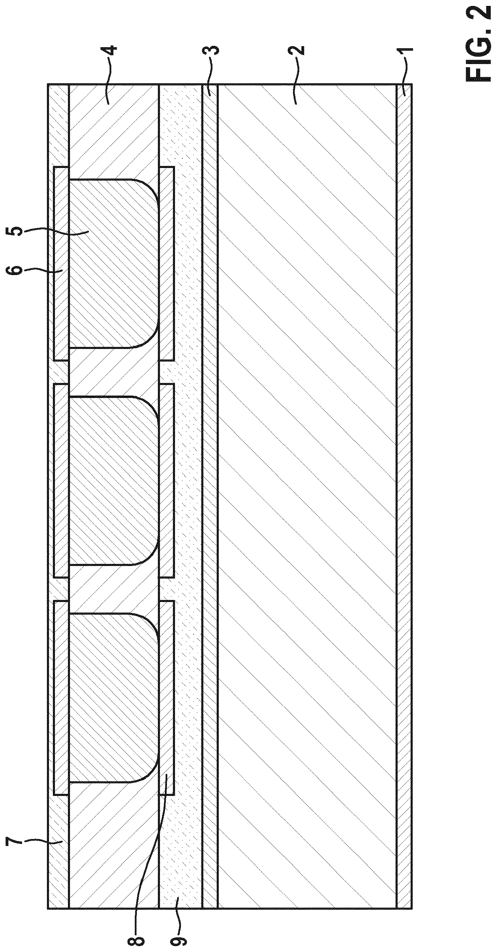

In der in Figur 2 gezeigten Ausführungsform des erfindungsgemäßen Ultraschallwandlers wird zunächst die flexible Leiterplatte bestehend aus dem Basismaterial 4, den piezoelektrischen Elementen 5 und den Leiterbahnen 6 und 8 hergestellt. Dies kann in einem für Leiterplatten typischen Produktionsformat (z.B. 300 x 450 mm oder auch noch grösser) erfolgen. Anschließend wird der Schallerzeuger 2 auf die Rückseite der Leiterplatte geklebt. Die Klebeschicht 9 kann überall oder nur an bestimmten Stellen elektrisch leitfähig sein, so dass ein elektrischer Kontakt zum Anschluss des Schallerzeugers 2 an eine der Leiterbahnen 8 der flexiblen Leiterplatte entsteht.

Dieser Aufbau hat insbesondere Vorteile, wenn die Leiterplatte noch andere Funktionen aufweist und der Schallerzeuger und der Detektor nur einen Teil der Leiterplatte ausmachen.

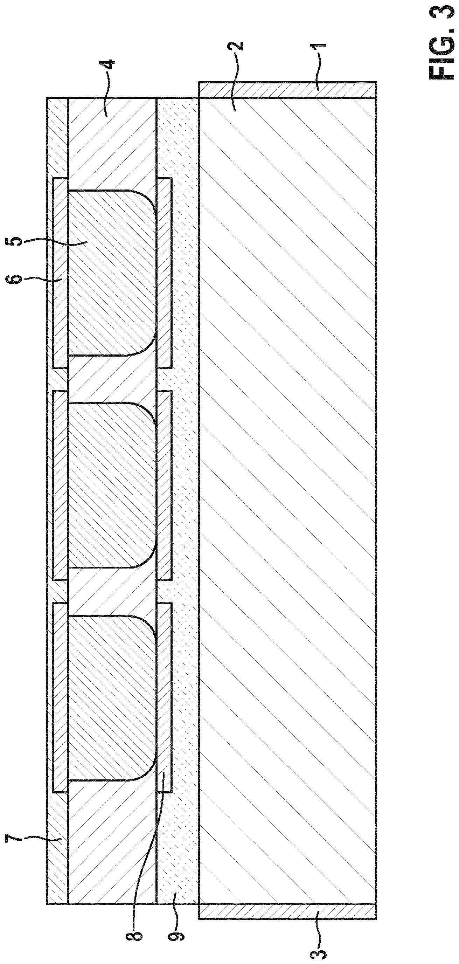

Wenn der Schallerzeuger 2 an den Stirnflächen elektrische Kontakte aufweist, wie Figur 3 gezeigt, kann eine höhere Komponente an Transversalwellen in das Körpergewebe eingekoppelt werden. Eine solche Anordnung ist nur bei Trennung des Detektors und des Schallerzeugers möglich.

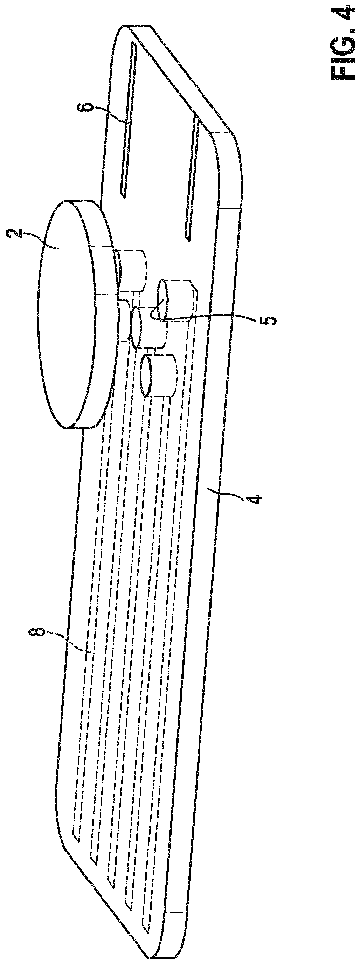

Ein vollständiger Aufbau ist schematisch Figur 4 dargestellt. Die piezoelektrischen Elemente 5 sind in die Leiterplatte 4 integriert und über die Leiterbahnen 8 angeschlossen. Die auf der Oberseite verlaufenden Leiterbahnen 6 sind nur schematisch angedeutet. Der Schallerzeuger 2 wird auf die Leiterplatte aufgeklebt. Die Reihenfolge der Darstellung ist hier umgekehrt als in den Querschnitten.

Diese Anordnung kann direkt in ein Gerät integriert werden. Die Leiterplatte kann auch direkt auf die Haut aufgelegt sein oder auf die Spitze oder den Ballon eines Katheters montiert sein. Auf der Oberseite des Schallerzeugers 2 können schallweiche Materialien angebracht sein, um eine höhere Energieübertragung in die andere Richtung zum Körpergewebe hin zu erreichen. Geeignete schallweiche Materialien sind Polymere im Allgemeinen und Elastomere im Besonderen.

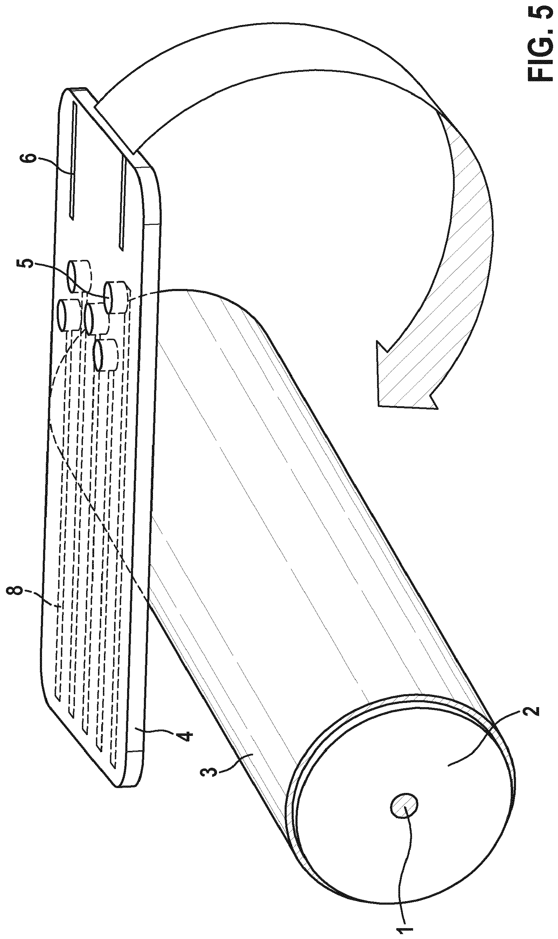

In einer anderen, in Figur 5 gezeigten Ausführungsform des erfindungsgemäßen Ultraschallwandlers wird die Leiterplatte 4 mit den integrierten Detektoren bzw. piezoelektrischen Elementen 5 über einen zylindrisch geformten Schallerzeuger 2 geklebt.

Bei diesem Konzept findet ein zylindrischer Wandler Verwendung. Eine Metallfläche 1 wird in diesem Fall zu einem Draht im Zentrum des piezoelektrischen Materials 2. Die Außenfläche des Zylinders 2 wird metallisiert 3. Im einfachsten Fall wird die Metallisierung 3 auf der Unterseite der Detektorfolie bzw. Leiterplatte 4 bereitgestellt.

Vorteilhafterweise kann mit der in Figur 5 gezeigten Ausführungsform eine Zylinderwelle erzeugte werden, die ein besseres Reflexionsverhalten im Gefäß ermöglicht und damit eine genauere Messung. Daher ist diese Anordnung insbesondere für die Anwendung in Kathetern interessant.

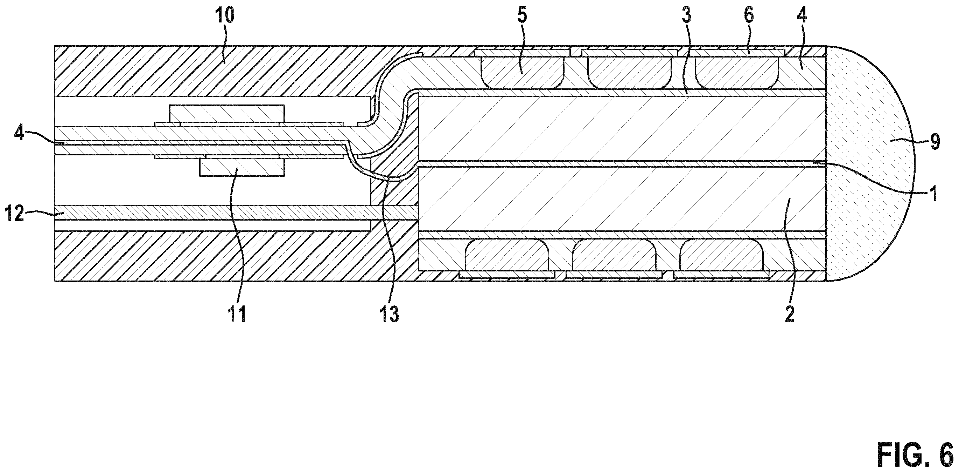

Ein solcherart ausgestalteter Katheter ist schematisch in Figur 6 dargestellt. Der Katheter umfasst dabei an seinem distalen Ende den erfindungsgemäßen Ultraschallwandler umfassend eine flexible Schaltung bzw. Leiterplatte 4 mit integriertem piezoelektrischen Elementen 5 als Ultraschallempfänger und einen koaxialen zylinderförmigen Ultraschallerzeuger 2. Der Katheter weist weiterhin eine Spitze 9 (als mechanischer Abschluss ohne weitere Funktion), einen Katheterschlauch 10, elektronische Komponenten 11, einen oder mehrere Führungsdrähte 12 (*guide wire*), sowie einen isolierten Draht 13 zum elektrischen Kontaktierung des Ultraschallerzeugers 2 (vorzugsweise mit Hochspannung). Der Draht 13 kann dabei an die flexible Leiterplatte 1 angeschlossen sein oder separat durch den Katheterschlauch 10 geführt werden. Die elektronischen Komponenten 11 sind dabei insbesondere für die Signalverarbeitung des Ultraschallempfängers 4,5 ausgebildet und können vorzugsweise auf der flexiblen Leiterplatte 4 montiert sein. Ein solcher Katheter weise vorzugsweise einen Durchmesser im Bereich von 1.5 mm bis 4 mm auf.

## Patentansprüche

1. Implantierbarer Ultraschallwandler mit
- einer Vorrichtung zum Erzeugen von Ultraschall (2),
- einer Vorrichtung zum Empfangen von Ultraschall (5), und
- einer Leiterplatte (4),
**dadurch gekennzeichnet,**
**dass** die Vorrichtung zum Erzeugen von Ultraschall (5) aus einem piezoelektrischen Polymer gebildet ist, welches in die Leiterplatte (4) integriert ist.

2. Implantierbarer Ultraschallwandler nach Anspruch 1, **dadurch gekennzeichnet, dass** die Vorrichtung zum Empfangen von Ultraschall (5) aus einer Vielzahl von piezoelektrischen Elementen (5) aus dem piezoelektrischen Polymer gebildet ist, wobei die Vielzahl von piezoelektrischen Elementen (5) in die Leiterplatte (4) integriert sind.

3. Implantierbarer Ultraschallwandler nach Anspruch 1 oder 2, **dadurch gekennzeichnet, dass** die Vorrichtung zum Erzeugen von Ultraschall (2) aus einer piezoelektrischen Material (2), insbesondere einer Keramik oder einem Kristall, gebildet wird.

4. Implantierbarer Ultraschallwandler nach einem der vorherigen Ansprüche, **dadurch gekennzeichnet**, die Vorrichtung zum Erzeugen von Ultraschall (2) an der Leiterplatte (4) angeordnet ist.

5. Implantierbarer Ultraschallwandler nach einem der vorherigen Ansprüche, **dadurch gekennzeichnet, dass** die Vorrichtung zum Erzeugen von Ultraschall (2) und die Vorrichtung zum Empfangen von Ultraschall (5) unabhängig voneinander elektrisch kontaktierbar sind.

6. Implantierbarer Ultraschallwandler nach einem der vorherigen Ansprüche, **dadurch gekennzeichnet, dass** die Leiterplatte (4) mindestens eine elektrische Leiterbahn (6, 8) umfasst, welche die Vorrichtung zum Empfangen von Ultraschall (5) elektrisch kontaktiert.

7. Implantierbarer Ultraschallwandler nach einem der vorherigen Ansprüche, **dadurch gekennzeichnet, dass** die Vorrichtung zum Erzeugen von Ultraschall (2) auf der Oberseite und der Unterseite jeweils eine Metallschicht (1,3) aufweist.

8. Implantierbarer Ultraschallwandler nach Anspruch 7, **dadurch gekennzeichnet, dass** die Vorrichtung zum Erzeugen von Ultraschall (2) mit der Oberseite an der Leiterplatte (4) angeordnet ist, wobei die Metallschicht (3) auf der Oberseite die Vorrichtung zum Empfangen von Ultraschall (5) elektrisch kontaktiert.

9. Implantierbarer Ultraschallwandler nach einem Ansprüche 1 bis 6, **dadurch gekennzeichnet, dass** die Vorrichtung zum Erzeugen von Ultraschall (2) an zwei einander gegenüberliegenden Seitenflächen jeweils eine Metallschicht (1, 3) aufweist.

10. Implantierbarer Ultraschallwandler nach einem der vorherigen Ansprüche, **dadurch gekennzeichnet, dass** die Leiterplatte (4) flexibel ist.

11. Implantierbarer Ultraschallwandler nach der Ansprüche 3 bis 9, **dadurch gekennzeichnet, dass** das piezoelektrische Material (2) im Wesentlichen im Form eines Hohlzylinders ausgebildet ist, wobei die Mantelseite des Hohlzylinders eine Metallschicht (3) aufweist, die den Durchgang des Hohlzylinders begrenzende Oberfläche mit Metall (1) beschichtet ist oder der Durchgang des Hohlzylinders mit Metall (1) ausgefüllt ist, und wobei die Leiterplatte (4) im Wesentlichen vollständig die Metallschicht (3) auf der Mantelseite umgibt.

12. Implantierbarer Ultraschallwandler nach einem der vorherigen Ansprüche, **dadurch gekennzeichnet, dass** das piezoelektrische Polymer (5) Polyvinylidenflourid oder ein Co-Polymer davon umfasst oder daraus besteht.

13. Implantierbarer Ultraschallwandler nach einem der vorherigen Ansprüche, **dadurch gekennzeichnet, dass** der implantierbare Ultraschallwandler zumindest teilweise mit einem biokompatiblen Lack oder Polymer (7), insbesondere ein Polydimethylsiloxan oder ein Polyurethan, beschichtet ist.

14. In den Körper einbringbares, insbesondere implantierbares, Medizingerät, aufweisend einen implantierbaren Ultraschallwandler nach einem der Ansprüche 1 bis 13.

15. In den Körper einbringbares Medizingerät nach Anspruch 14, **dadurch gekennzeichnet, dass** das Medizingerät als ein aktives Implantat, ein Sensor, ein Loop-Recorder oder ein Katheter ausgebildet ist.
